Europäisches Patentamt

**European Patent Office**

**Office européen des brevets**

(19)

(11) Publication number: **0 146 903**
**A2**

(12)

# EUROPEAN PATENT APPLICATION

(21) Application number: **84115623.5**

(22) Date of filing: **17.12.84**

(51) Int. Cl.⁴: **C 12 N 15/00**
**C 12 P 21/02, A 61 K 45/02**
**C 07 H 21/04, C 07 K 15/26**

(30) Priority: **19.12.83 US 562639**
**21.12.83 US 564066**

(43) Date of publication of application:
**03.07.85 Bulletin 85/27**

(84) Designated Contracting States:
**AT BE CH DE FR GB IT LI NL SE**

(71) Applicant: SCHERING CORPORATION
2000 Galloping Hill Road
Kenilworth, New Jersey 07033(US)

(72) Inventor: Leibowitz, Paul Jason
185 Prospect Avenue
Hackensack New Jersey 07601(US)

(72) Inventor: Ryan, Michael Joseph
34 Northwood Drive
West Milford New Jersey 07480(US)

(74) Representative: Ritter, Stephen David et al,
Mathys & Squire 10 Fleet Street
London EC4Y 1AY(GB)

(54) A novel hybrid interferon species.

(57) The invention provides a novel hybrid interferon species that comprises a chain of 161 and/or 162 amino acids. The hybrid is novel not only for its new structure, but also because the hybrid comprises a shortened or truncated segment of alpha interferon and hence is an entirely new interferon species that does not occur in nature.

The invention further provides pharmaceutical compositions containing said novel interferons;

DNA sequences coding for said novel interferons;

vectors containing and capable of expressing said DNA sequences;

hosts transformed with said vectors;

and methods for producing these products.

EP 0 146 903 A2

## A NOVEL HYBRID INTERFERON SPECIES

This invention relates to a novel alpha interferon-type protein having interesting antiviral and anti-tumor activities. This protein can be produced by culturing an organism containing expressible recombinant DNA that codes for the novel alpha interferon-type protein.

The antiviral and anti-tumor activity of various interferons is currently being explored. Recently, hybrid interferons that do not naturally occur in nature have been prepared; see for example, United States Patent No. 4,414,150, issued November 8, 1983.

However, such hybrid interferons contain complete portions of natural interferon sequences without deletions, i.e., the segments that are joined together are complete.

The present invention provides an advance in that the novel alpha-interferon-type protein contains a shortened or truncated segment or sequence, i.e. a "sub-segment", of a natural alpha-interferon, joined to a segment (or sequence) from another alpha-interferon; and this novel alpha-interferon-type protein surprisingly shows enhanced activity.

0146903

-2-

We have found that, when this novel hybrid is formed specifically corresponding to a truncated alpha-2 segment and an alpha-1 segment, the resultant species has a very desirable biological profile.

As is well recognized in the art, the codon ATG has a special meaning as initiation codon, i.e. coding for the amino acid methionine as the first amino acid in a protein comprising a chain of amino acids. Sometimes the host in which this protein is produced (e.g. by recombinant DNA techniques) will cleave this amino-terminal methionine off the protein and sometimes not, so that two closely related species can be produced. According to the present invention, the species lacking methionine at the amino terminus will have 161 amino acids and start with glutamine, and the species starting with methionine at the amino-terminus will have 162 amino acids.

The new interferon species of this invention features a "sub-segment" defined as a delta-4 alpha-2 (Bgl II-1) derived from an alpha-2 sequence, joined to a segment defined as (Bgl II) alpha-1 derived from an alpha-1 sequence. It surprisingly shows heightened antiviral and anti-tumor activity.

The proteins of the present invention are characterized as having an antiviral activity of at least $1 \times 10^7$ units/mg as determined by the cytopathic effect inhibition assay employing EMC virus and human foreskin cells (FS-71) performed essentially as described in a publication by Familletti et al. (Reference 1), using as a standard the NIH/WHO uncloned leukocyte interferon standard 69/19.

For the purposes of clarification and definition, the gene coding for the novel alpha interferon-type proteins is composed of a portion of the amino terminal nucleotide coding sequence from the mature human alpha-2 interferon gene up to the first Bgl II site - hence the phraseology "Bgl II-1" - joined to the complete carboxy terminal nucleotide coding sequence from the mature alpha-1 interferon gene at the only Bgl II site therein. The portion of the human alpha-2 interferon gene used to construct the gene that codes for the invention lacks the first 12 nucleotides normally found in the sequence coding for the mature human alpha-2 interferon, and therefore does not contain genetic information that would code for these first four amino-terminal amino acids (CYS-ASP-LEU-PRO); hence we use the phraseology "delta-4" to reflect this feature of the sub-segment.

The portion of the human alpha-1 interferon gene used to construct the gene that codes for the invention is composed of all nucleotides found downstream from the only Bgl II site found in the mature human alpha-1 interferon coding sequence; hence the phraseology "(Bgl II) alpha-1".

Therefore, we use the phraseology "delta-4 alpha-2 (Bgl II-1)" to describe the nucleotide coding sequence obtained from the mature human alpha-2 interferon gene that was used to construct the gene that codes for the invention, and we use the phraseology "(Bgl II) alpha-1" to describe the nucleotide coding sequence obtained from the mature human alpha-1 interferon gene that was used to construct the gene that codes for the invention. Furthermore, we also define the protein sub-segment and protein segment with this phraseology.

For the purposes of definition, mature human alpha-1 interferon is defined as having the amino acid sequence shown below:

CYS ASP LEU PRO GLU THR HIS SER LEU ASP ASN ARG ARG THR
LEU MET LEU LEU ALA GLN MET SER ARG ILE SER PRO SER SER
CYS LEU MET ASP ARG HIS ASP PHE GLY PHE PRO GLN GLU GLU
PHE ASP GLY ASN GLN PHE GLN LYS ALA PRO ALA ILE SER VAL
LEU HIS GLU LEU ILE GLN GLU ILE PHE ASN LEU PHE THR THR
LYS ASP SER SER ALA ALA TRP ASP GLU ASP LEU LEU ASP LYS
PHE CYS THR GLU LEU TYR GLN GLN LEU ASN ASP LEU GLU ALA
CYS VAL MET GLN GLU GLU ARG VAL GLY GLU THR PRO LEU MET
ASN ALA ASP SER ILE LEU ALA VAL LYS LYS TYR PHE ARG ARG
ILE THR LEU TYR LEU THR GLU LYS LYS TYR SER PRO CYS ALA
TRP GLU VAL VAL ARG ALA GLU ILE MET ARG SER LEU SER LEU
SER THR ASN LEU GLN GLU ARG LEU ARG ARG LYS GLU.

The mature human alpha-2 interferon is defined as having the amino acid sequence shown below:

CYS ASP LEU PRO GLN THR HIS SER LEU GLY SER ARG ARG THR
LEU MET LEU LEU ALA GLN MET ARG ARG ILE SER LEU PHE SER
CYS LEU LYS ASP ARG HIS ASP PHE GLY PHE PRO GLN GLU GLU
PHE GLY ASN GLN PHE GLN LYS ALA GLU THR ILE PRO VAL LEU
HIS GLU MET ILE GLN GLN ILE PHE ASN LEU PHE SER THR LYS
ASP SER SER ALA ALA TRP ASP GLU THR LEU LEU ASP LYS PHE
TYR THR GLU LEU TYR GLN GLN LEU ASN ASP LEU GLU ALA CYS
VAL ILE GLN GLY VAL GLY VAL THR GLU THR PRO LEU MET LYS
GLU ASP SER ILE LEU ALA VAL ARG LYS TYR PHE GLN ARG ILE
THR LEU TYR LEU LYS GLU LYS LYS TYR SER PRO CYS ALA TRP
GLU VAL VAL ARG ALA GLU ILE MET ARG SER PHE SER LEU SER
THR ASN LEU GLN GLU SER LEU ARG SER LYS GLU.

The invention relates to a new species of interferon or protein employing an abbreviated segment or "sub-segment" designated as delta-4 alpha-2 (Bgl II-1) derived from an alpha-2 sequence and joined to a segment designated as (Bgl II) alpha-1 derived from an alpha-1 sequence.

For the further purpose of definition, the term "segment" shall define a portion of an interferon chain of amino acids that is bounded at one end by a naturally occurring sequence found at one end of the mature molecule.

For the further purpose of definition, the terms "abbreviated segment" and "sub-segment" shall define a portion of an interferon chain of amino acids that is not bounded at either end by either of the naturally occurring sequences found at the ends of the mature molecule and, therefore, is a shortened portion of an interferon amino acid chain that does not comprise the full complement of amino acids that would normally be found at one end in a "segment" of interferon as defined above.

The invention can be further described as comprising a protein having a sequence of 161 or 162 amino acids as defined below:

$(MET)_X$ GLN THR HIS SER LEU GLY SER ARG ARG THR LEU MET
LEU LEU ALA GLN MET ARG ARG ILE SER LEU PHE SER CYS LEU
LYS ASP ARG HIS ASP PHE GLY PHE PRO GLN GLU GLU PHE GLY
ASN GLN PHE GLN LYS ALA GLU THR ILE PRO VAL LEU HIS GLU
MET ILE GLN GLN ILE PHE ASN LEU PHE THR THR LYS ASP SER
SER ALA ALA TRP ASP GLU ASP LEU LEU ASP LYS PHE CYS THR
GLU LEU TYR GLN GLN LEU ASN ASP LEU GLU ALA CYS VAL MET
GLN GLU GLU ARG VAL GLY GLU THR PRO LEU MET ASN ALA ASP
SER ILE LEU ALA VAL LYS LYS TYR PHE ARG ARG ILE THR LEU
TYR LEU THR GLU LYS LYS TYR SER PRO CYS ALA TRP GLU VAL
VAL ARG ALA GLU ILE MET ARG SER LEU SER LEU SER THR ASN
LEU GLN GLU ARG LEU ARG ARG LYS GLU

wherein X can be either 0 or 1.

The invention also comprises a mixture of these first and second proteins having 161 and 162 amino acids, respectively, the first protein starting with glutamine and the second protein having the amino acid sequence of the first protein but differing from

-6-

the first protein in that methionine precedes the complete amino acid sequence of the first protein.

The protein or protein mixture is characterized as having an antiviral activity of at least 1 x 10$^7$ units/mg as determined by the cytopathic effect-inhibition assay employing EMC virus and human foreskin cells (FS-71) performed essentially as described in a publication by Familletti et al. (Reference 1), using as a standard the NIH/WHO uncloned leukocyte interferon standard 69/19.

The resulting protein or protein mixture comprises a new interferon species that is not a naturally occurring alpha interferon.

The invention further comprises a DNA sequence coding for said proteins and preferably having the sequence:

(ATG) CAA ACC CAC AGC CTG GGT AGC AGG AGG ACC TTG ATG
CTC CTG GCA CAG ATG AGG AGA ATC TCT CTT TTC TCC TGC TTG
AAG GAC AGA CAT GAC TTT GGA TTT CCC CAG GAG GAG TTT GGC
AAC CAG TTC CAA AAG GCT GAA ACC ATC CCT GTC CTC CAT GAG
ATG ATC CAG CAG ATC TTC AAC CTC TTT ACC ACA AAA GAT TCA
TCT GCT GCT TGG GAT GAG GAC CTC CTA GAC AAA TTC TGC ACC
GAA CTC TAC CAG CAG CTG AAT GAC TTG GAA GCC TGT GTG ATG
CAG GAG GAG AGG GTG GGA GAA ACT CCC CTG ATG AAT GCG GAC
TCC ATC TTG GCT GTG AAG AAA TAC TTC CGA AGA ATC ACT CTC
TAT CTG ACA GAG AAG AAA TAC AGC CCT TGT GCC TGG GAG GTT
GTC AGA GCA GAA ATC ATG AGA TCC CTC TCT TTA TCA ACA AAC
TTG CAA GAA AGA TTA AGG AGG AAG GAA [TAA]

wherein the initiation codon ATG is enclosed in round brackets and a STOP-codon TAA is enclosed in square brackets. The invention further comprises DNA sequences coding for said proteins but differing from the above sequence according to the degeneracy of the genetic code.

The invention also comprises a cloning vector (e.g. a plasmid) including a DNA sequence as above described, operatively linked to an expression control sequence, and a host, e.g. bacterium, yeast or animal cell, transformed with said cloning vector.

Tests with the above protein or proteins have shown a high activity against viruses and tumors, as compared to standard commercial alpha-2 interferon. Of particular interest, the above protein or proteins have demonstrated favorable activity against adenovirus, such as adenovirus-1, and ovarian and cervical carcinomas.

The protein or proteins of the invention have been genetically engineered from two different interferon coding sequences by joining a coding sequence for a sub-segment of mature alpha-2 interferon with a coding sequence for a segment of mature alpha-1 interferon.

The 161 (or 162) amino acid chain of these proteins is theoretically believed to have sulphur-sulphur bonding between the cysteine residue at position 25 and the cysteine residue at position 134. (In this numbering, the methionine residue optionally present as first amino acid when X is 1 has been given position zero.) It is also believed that the biological activity of the inventive protein is related to this disulfide bond, and may also result from the character of the uniquely combined sequence provided for by the segment and sub-segment derived respectively from the alpha-1 and alpha-2 mature coding sequences.

The general method for making this novel alpha interferon-type protein was to prepare a hybrid interferon gene that codes for the novel alpha interferon-type protein. This first entailed the joining of a group of promoters with translation initiation signals to the start of the mature alpha-2 interferon coding sequence. Subsequently, DNA fragments in which the promoters had been joined to the alpha-2 amino terminal coding sequences (up to the first Bgl II restriction site) were identified, isolated, and ligated to the carboxy terminal coding sequences of the alpha-1 interferon gene that immediately followed the Bgl II site found in the mature alpha-1 coding sequences.

The actual experiments performed can be grouped into six broad activity areas and constitute a feature of the invention: (1) construction of a derivative of an alpha-1 interferon plasmid, in particular the plasmid Hif-2h (Reference 2) that has only one Eco RI site, rather than two; (2) the assembly of a group (or family) of promoters that end in an ATG translation initiation codon and differ in the number of nucleotides between the "Shine-Dalgarno" sequence and this initiation codon; (3) the joining of this group of promoters to the amino-terminal coding sequence of the alpha-2 interferon gene; (4) the isolation of DNA fragments which included the promoter regions ligated to the amino-terminal coding sequence of the alpha-2 interferon gene; (5) the joining of these promoter-containing fragments to the carboxy-terminal alpha-1 interferon coding sequences; and (6) the screening of bacterial clones for the production of active hybrid interferon, so that bacterial clones containing appropriate recombinant molecules can be isolated.

The method used for transforming E. coli was essentially as described (Reference 13). Restriction enzymes, T4 DNA ligase, and any other DNA modifying
enzymes obtained from New England Biolabs and Bethesda
Research Laboratories were used generally according to
the manufacturer's recommendations. Methods for
recovering DNA from gels were generally according to
those reviewed by H. O. Smith: "Recovery of DNA from
gels," Methods in Enzymology, Vol. 65, Part 1, edited
by L. Grossman and K. Moldave, Academic Press, N.Y.
(1980), pages 371-380. Protocols for methods and
procedures not specifically described below (or
essentially equivalent substitutes) can be found in a
number of available "recipe books": for example see
Reference 12. Each of these above six activity areas
is described in more detail below.

1.    Construction of a derivative of the Alpha-1
Interferon Plasmid Hif-2h that has only one Eco RI
site, by Deletion of a non-coding region beyond the end
of the alpha-1 Interferon gene. The alpha-1 interferon
coding sequence is contained in the plasmid designated
Hif-2h (Reference 2) which has two Eco RI sites. An
aliquot of this plasmid DNA was subjected to digestion
with restriction endonuclease Eco RI under conditions
that would be expected to produce some partial
digestion products. The exposed ends were trimmed with
nuclease S1 followed by a total digestion with Bam
HI. The desired DNA fragment, which carried the alpha-
1 interferon gene up to the former Eco RI site in the
non-coding region as well as the amino-terminal coding
end of the tetracycline resistance gene, was identified
by its mobility on an agarose gel relative to markers
of known size, and was isolated. In parallel, pBR322
(Reference 3) DNA was digested with Pst I followed by
treatment with nuclease S1 and finally Bam HI
digestion. In this case, the fragment that was isolated carried the origin of replication for pBR322 as well as the carboxy terminal coding sequences for the tetracycline resistance gene. The above two fragments were enzymatically joined with T4 DNA ligase, and the recombinant DNA was used to transform the E. coli strain 294 (Reference 4). Plasmid DNA from tetracycline-resistant transformants was subjected to restriction enzyme analysis to verify the structure, in which there is only one Eco RI site, that is upstream of the alpha-1 interferon coding sequence.

2. Assembly of a group of promoters that end in an ATG translation initiation codon and differ in the number of nucleotides between the "Shine-Dalgarno" sequence and this ATG translation initiation codon. A plasmid was constructed in which there was a unique Eco RI restriction site approximately 30 base pairs upstream from the sequence ...CCTCGCCCTTTGCTTTACTGATGGTCC... (obtained from the "leader" coding sequence of the alpha-1 interferon gene in the plasmid Hif-2h). This plasmid was constructed by cloning a forty base-pair fragment of DNA (i.e., the Hae III/Pvu II restriction fragment found within the coding region for the leader sequence for the human alpha-1 interferon gene of Hif-2h) between the Hind III and Pvu II sites of pBR322 in the orientation that regenerated the Pvu II site.

Aliquots of this plasmid DNA were first linearized by enzymatic digestion at the unique Eco RI restriction site. Then, varying numbers of nucleotides between this point and the above (underlined) ATG codon were removed by different extents of nuclease digestion using Exonuclease III followed by nuclease S1 or, in another experiment, with nuclease Bal 31 generally following published procedures (for example, References 6 and 12) and/or the manufacturer's recommendations.

These trimmed DNA molecules were ligated to a nominally
125 base pair lac UV5 promoter (derived from pKB252
[Reference 5]) fragment that carries the "Shine-
Dalgarno" sequence for initiating translation.  The
sequence of this promotor fragment is as follows:
5'-GAATTCCAGTGAATCCGTAATCATGGTCATAGCTCACTCATTAGGCA
    CCCCAGGCTTTACACTTTATGCTTCCGGCTCGTATAATGTGTGGAATTGTGA
    GCGGATAACAATTTCACACAGGAAACAG-3'
The resulting population of recombinant DNA molecules
was used to transform the E. coli strain 294 to
ampicillin-resistance.  Plasmid DNA was isolated from a
population of these ampicillin-resistant bacteria and
used for the isolation of a family of lac promoters
that should carry a promoter, operator and Shine-
Dalgarno sequence, and end with the translation
initiation codon ATG.  The members of this family
differ from one another in the number of nucleotides
between the Shine-Dalgarno sequence and the ATG codon
and, therefore, are expected to have different
efficiencies of initiating translation (References 4,
6, and 7).

An aliquot (58 µg) of this DNA (containing
the family of lac promoters) isolated from a non-
methylating E. coli strain was digested with the
restriction endonuclease Ava II (which recognizes the
nucleotide sequence GG(A/T)CC and cleaves each strand
of the DNA between the G residues) in a reaction volume
of 250 ul.  After this digestion, the sample was placed
on ice, 30 µl of 10X Sl salts (1.5M NaCl, 0.5M sodium
acetate pH 4.0, 0.06M $ZnSO_4$), 20 µl 5M NaCl, and 2 µl
(200 units) of nuclease Sl (Sigma catalog N-5255) were
added, and the mixture was incubated at 11°C for ten
minutes.  To this reaction 0.5 ml of (0.3M sodium
acetate, 0.01M EDTA, 0.2M tris HCl pH 9.5) was added,

and the DNA was precipitated by addition of 2.5 volumes of ethanol. The pelleted DNA was resuspended in an appropriate buffer and digested with Eco RI. The products of this reaction were analyzed on an acrylamide gel, and the population of DNA fragments that carried the promoter and translation initiation sequences was observed as a broad DNA band of (approximately) >125 base pairs in length. Within this group was a collection of fragments that have a cleaved Eco RI site at one end and an ATG codon that serves as a translation initiation signal at the other end (a flush end). This entire collection of DNA fragments was isolated from the acrylamide gel to be joined to the amino terminal coding end of the alpha-2 interferon gene in the next steps, described below.

3.    Joining this group of promoters to the amino-terminal coding sequence of the alpha-2 interferon gene. The starting material for this step included an alpha-2 interferon gene in which a Hind III restriction site had been placed at the start of the mature alpha-2 coding sequence creating the nucleotide sequence AAGCTTGT.... The underlined codon (TGT) codes for cysteine which is the first amino acid of the mature alpha-2 interferon species. A description of this type of construction is presented in Reference 8 (Figure 9, Structures 1-3). This plasmid containing the alpha-2 interferon gene also had a unique Eco RI site upstream of this unique Hind III site.

Therefore, sequential digestions of this DNA with Hind III, nuclease S1, and Eco RI yields a vector into which the lac promoters (prepared in step 2, above) could be joined in a predetermined orientation. More specifically, fifty micrograms of this plasmid containing the alpha-2 interferon gene were digested to completion with Hind III in a 350 μl reaction. The sample was transferred to ice and 50 μl of 10X Sl salts, 35 μl of 5M NaCl, and 2 μl (200 units) of nuclease Sl were added. This mixture was incubated at 11°C for 10 minutes after which 0.41 ml of a solution containing 0.3M sodium acetate, 0.01M EDTA and 0.2M tris HCl pH 9.5 was added and the DNA was precipitated by the addition of 2.5 volumes of ethanol. After this DNA was resuspended, it was digested with Eco RI, after which the enzyme was inactivated by heating at 65°C. The DNA was again precipitated with ethanol and finally resuspended and enzymatically joined by T4 DNA ligase to the collection of promoters isolated above. The recombinant DNA molecules were then used to transform E. coli strain D1210 (References 9 and 13) to ampicillin resistance.

4. Isolation of DNA fragments including the promoter (lac promoter) ligated to the amino-terminal coding sequence of the mature alpha-2 interferon gene.

Plasmid DNA was isolated from an entire population of ampicillin-resistant transformants of D1210 obtained in step 3 and a 60 microgram aliquot was subjected to digestion with the restriction endonucleases Eco RI and Bgl II. The products of this reaction were electrophoresed on an 8% polyacrylamide gel. As expected, there was a broad, fuzzy, band of fragments approximately 330 ± 20 base pairs in length. These fragments, therefore, were expected to contain lac transcription and translation initiation signals fused to the amino terminal coding region (as far as the first Bgl II site) of the alpha-2 interferon gene. The broad band encompassing this group of fragments was isolated from the gel.

5.   Forming the alpha-2/alpha-1 hybrid interferon gene
by joining these promoter-containing fragments to the
carboxy-terminal alpha-1 interferon coding sequences.
An aliquot of plasmid DNA containing the alpha-1
interferon gene (constructed in step 1) was digested
with the restriction endonucleases Eco RI and Bgl II,
and the larger of the two fragments (which carries the
carboxy terminal coding region for the alpha-1
interferon as well as the gene for the tetracycline
resistance) was isolated after agarose gel
electrophoresis.  This larger DNA fragment was then
ligated to the population of lac promoter fragments
isolated in step 4.

6.   Isolating and characterizing a clone expressing a
hybrid interferon with antiviral activity by screening
bacterial clones for the production of active hybrid
interferon.  The ligated DNA molecules formed in the
previous step (which should have the lac regulatory
elements fused to an alpha-2/alpha-1 hybrid interferon
gene) were used to transform the E. coli strain 294 to
tetracycline resistance.  Individual colonies were
picked and grown, and extracts were prepared.  These
extracts were assayed for the presence of interferon
activity using procedures essentially as described in
the literature (References 1 and 10).

Plasmid DNA was isolated from the clone
producing the highest level antiviral activity.  The
junction between the lac promoter and the amino
terminal coding end of this hybrid interferon coding
sequence was then subjected to DNA sequence analysis.
This revealed, unexpectedly, that the coding sequence
for the first four amino acids had been removed during
the constructions described above, presumably due to
the "fraying" activity known to be associated occasionally with the single-strand-nuclease S1 on the ends of linear duplex DNA molecules (Reference 6). A partial nucleotide sequence from this region is presented below:

```
                 (fmet) Gln Thr His Ser Leu
    ...AGGAAACAGACTG  ATG  CAA ACC CAC AGC CTG...
```

An alternative, Preferred Method for the Construction and Expression of a Delta-4 Alpha-2 (Bgl II-1)/(Bgl II) Alpha-1 Hybrid Interferon Gene

A further feature of the invention comprises the following process for the preparation of a plasmid containing a gene coding for and capable of expressing the novel alpha-type interferons defined herein, and of a host transformed with such plasmid, which comprises:

a) Isolating from a plasmid containing the alpha-2 interferon gene a fragment (up to about 300 base pairs long) extending from a restriction site preceding the gene to the Pvu II restriction site within that gene;

b) Annealing the coding strand of this fragment to a synthetic oligonucleotide (which can be, for example, about 15 base pairs long), the sequence of which starts with the first nucleotide residue of the fifth codon of the mature alpha-2 interferon coding sequence;

c) Filling in the non-coding strand in the 5'→3'-direction and eating away the coding strand in the 3'→5'-direction and digesting the resulting double strand DNA fragment with Bgl II;

d) Digesting with Eco RI an alpha-1 interferon plasmid that has only one Eco RI site at a position upstream of the coding sequence for the alpha-1 interferon, filling it in and digesting it with Bgl II;

-16-

e) Ligating the larger fragment from this second plasmid to the amino terminal coding region of alpha-2 interferon DNA derived from step c) and thereby recreating the Eco RI site);

f) Transforming a suitable host; and

g) Modifying the plasmid to make the gene expressible.

A suitable host for step f) is E. coli; this can be cultivated to multiply the plasmid so that the step of modifying it in step g) is easier. In step g) one takes advantage of the unique Eco RI site now available: The plasmid is digested with Eco RI, both single strand ends are removed so that flush ends are generated, and a flush-ended fragment containing in sequence a promoter, a ribosome binding site and (at its end) a translation initiation codon is ligated in the gap to precede and control the hybrid alpha-2-alpha-1 interferon gene, and the plasmid is recircularised.

This method is more flexible than the first method herein described, since it will allow the incorporation of different genetic regulatory sequences. A preferred embodiment is described below:

The starting materials for this construction preferably include a derivative of the alpha-2 interferon gene with a Hind III restriction endonuclease site at the start of the mature coding sequence as mentioned above and described in the literature (Reference 8) and the aforementioned (Bgl II) alpha-1 fragment. The procedure comprises the isolation from this plasmid containing the alpha-2 interferon gene of a (nominally) 276 base pair Hind III/ Pvu II fragment that would span the region of interest. This fragment is then annealed with a

-17-

synthetic oligonucleotide (which can be custom-synthesized commercially), for example, fifteen nucleotides long having the following sequence:

5'-CAAACCCACAGCCTG-3'.

The kinased oligonucleotide should be annealed to a template (which should be a heat denatured Hind III/Pvu II DNA fragment described above which includes a portion of the nucleotide coding sequence for the mature human alpha-2 interferon gene). The synthetic oligonucleotide will form a duplex DNA structure starting with the first nucleotide of the codon for the fifth amino acid of the mature alpha-2 interferon. The concerted action of the polymerase and 3'-5' exonuclease activities of the Klenow fragment of the E. coli DNA polymerase I, in the presence of the four deoxynucleoside triphosphates, followed by digestion with Bgl II, will release the amino terminal end (up to the first Bgl II site) of alpha-2 interferon, but this will lack codons for the first four amino acids. The desired fragment can be readily visualized by autoradiography if, for example, the oligonucleotide has been kinased with gamma-$^{32}$P-ATP. Protocols for each of these steps can be found in the literature (Reference 11).

The alpha-1 fragment recipient plasmid described above can be digested with Eco RI and then "filled in" with, for example, the Klenow fragment of DNA polymerase in the presence of the four dNTP's and then digested with Bgl II. Removal of the 5'-terminal phosphates at this point is recommended to decrease the background in the following steps. The larger fragment produced by these manipulations is then ligated to the amino terminal coding region of alpha-2 isolated in the step above. The recombinant DNA formed and isolated after transformation of a suitable E. coli host and selection of tet$^R$ clones will, therefore, have an Eco RI site immediately before the start of the delta-4 alpha-2 (Bgl II-1/Bgl II) alpha-1 interferon structural gene. This gene can be expressed in a number of ways: for example, the molecule could be digested by Eco RI followed by any one of a number of single strand specific nucleases (Reference 12) to generate a flush end against which one could ligate a flush-ended fragment containing a promoter and ribosome binding site, and ending with a translation initiation codon. Productive clones will be those in which the regulatory elements are in the correct orientation relative to the coding sequence for the novel interferon.

The invention further comprises pharmaceutical compositions comprising, as active ingredient, a novel protein or protein mixture as hereinabove defined, together with a pharmaceutical carrier or excipient.

The novel interferon is preferably administered parenterally (e.g., intravenously, subcutaneously, intramuscularly) to patients, using methods and dosages similar to those used for human alpha-2 interferon. It is presumed that the novel interferon species will be useful when applied topically (e.g., corneal applications to treat susceptible viral eye infections).

Topical dosages from $10^5$-$10^8$ U/M$^2$ body surface area/day will be appropriate but the attending physician will determine the correct dose dependent upon the particular patient and the precise condition under treatment. The following formulation can be prepared as described in European Patent Application 82481, published June 29, 1983:

-19-

## EXAMPLE

Solution for Lyophilisation (1000 vials; 1 ml. per vial)

## Formula

| | |
|---|---|
| Interferon | $7.5 \times 10^{10}$ I.U. |
| Disodium Hydrogen Phosphate, Anhydrous, USP, Reagent | 2.27 grams per liter. |
| Sodium Dihydrogen Phosphate, USP | 0.55 grams per liter. |
| Glycine, USP | 20.0 grams per liter. |
| Human Albumin, USP | 1.0 grams per liter. |
| Water for Injection, USP q.s. ad | 1.0 liter |

The Interferon may be either hybrid interferon according to the present invention or a mixture of those interferons.

-20-

## APPENDIX: REFERENCES

1. Familletti, Philip C., Sara Rubenstein, and Sidney Pestka: Methods in Enzymology, 78:387-394 (1981), Academic Press Inc.

2. Mantei, N., M. Schwarzstein, M. Streuli, S. Panem, S. Nagata, and C. Weissmann: The nucleotide sequence of a cloned human leukocyte interferon cDNA. Gene 10:1-10 (1980).

3. Bolivar, F., R. L. Rodriguez, P. J. Green, M. C. Betlach, H. L. Heyneker, H. W. Boyer, J. H. Crosa, and S. Falkow: Construction and characterization of new cloning vehicles. II. A multi-purpose cloning system. Gene 2:95 (1977).

4. Backman, K. and M. Ptashne: Maximizing Gene Expression on a Plasmid Using Recombination in Vitro. Cell 13:65-71 (1978).

5. Backman, K., M. Ptashne, and W. Gilbert: Construction of plasmids carrying the CI gene of bacteriophage lambda. Proc. Nat. Acad. Sci. 73:4174-4178 (1976).

6. Roberts, Thomas M. and Gail D. Lauer: Maximizing Gene Expression on a Plasmid Using Recombination in Vitro. Methods in Enzymology, 68:473-482, Academic Press Inc. (1979).

7. Roberts, Thomas M., Raymond Kacich, and Mark Ptashne: A general method for maximizing the expression of a cloned gene. Proc. Nat. Acad. Sci., Vol. 76, No. 2, pp. 760-764 (1979).

8. Weissmann, C.: The Cloning of Interferon and Other Mistakes; in Interferon, 1981, Volume 3, p. 101, edited by Ion Gresser, Academic Press, N.Y. (1979).

9. Sadler, J. R., M. Tecklenburg, and J. L. Betz: Plasmids Containing Many Tandem Copies of a Synthetic Lactose Operator. Gene 8:279-300 (1980).

10. Nagata, S., H. Taira, A. Hall, L. Johnsrud, M. Streuli, J. Ecsodi, W. Boll, K. Cantell, and C. Weissmann: Synthesis in E. coli of a polypeptide with human leukocyte interferon activity. Nature 284:316-320 (1980).

11. Goeddel, David V., H. Michael Shepard, Elizabeth Yelverton, David Leung, and Roberto Crea: Synthesis of human fibroblast interferon by E. coli. Nucleic Acids Res., Vol. 8, No. 18, pp. 4057-4074 (1980).

12. Protocols for methods and procedures not specifically described above or essentially equivalent substitutes can be found in a number of available "recipe books." For example: T. Maniatis, E. F. Fritsch, and J. Sambrook, "Molecular Cloning - A Laboratory Manual" (1982), published by the Cold Spring Harbor Laboratory, Cold Spring Harbor, New York.

13. Dagert, M. and S. D. Ehrlich: Prolonged Incubation in Calcium Chloride Improves the Competence of Escherichia coli Cells. Gene 6:23-28 (1979).

CLAIMS

1.    A protein having an amino acid sequence of 161 or 162 amino acids depending on the optional addition of methionine attached to a first amino acid in said sequence, said sequence including different portions of two different naturally occurring alpha interferons as joined discrete sub-sequences thereof defined and arranged as a delta-4 alpha-2 (Bgl II-1) sub-segment preceding a (Bgl II) alpha-1 segment, wherein said protein is a chain of amino acids that does not naturally occur as an alpha interferon.

2.    A protein comprising a sequence of 161 or 162 amino acids as defined below:

(MET)$_X$ GLN THR HIS SER LEU GLY SER ARG ARG THR LEU MET LEU LEU ALA GLN MET ARG ARG ILE SER LEU PHE SER CYS LEU LYS ASP ARG HIS ASP PHE GLY PHE PRO GLN GLN GLU PHE GLY ASN GLN PHE GLN LYS ALA GLU THR ILE PRO VAL LEU HIS GLU MET ILE GLN GLN ILE PHE ASN LEU PHE THR THR LYS ASP SER SER ALA ALA TRP ASP GLU ASP LEU LEU ASP LYS PHE CYS THR GLU LEU TYR GLN GLN LEU ASN ASP LEU GLU ALA CYS VAL MET GLN GLU GLU ARG VAL GLY GLU THR PRO LEU MET ASN ALA ASP SER ILE LEU ALA VAL LYS LYS TYR PHE ARG ARG ILE THR LEU TYR LEU THR GLU LYS LYS TYR SER PRO CYS ALA TRP GLU VAL VAL ARG ALA GLU ILE MET ARG SER LEU SER LEU SER THR ASN LEU GLN GLU ARG LEU ARG ARG LYS GLU

wherein X can be either 0 or 1, and further wherein said protein has an antiviral activity of at least about 1 x 10$^7$ units/mg as determined by the cytopathic effect-inhibition assay.

3.    A mixture of proteins defined in claim 1 or claim 2.

4.    Pharmaceutical compositions comprising, as active ingredient, a protein or protein mixture as claimed in

0146903

-23-

any of claims 1 to 3, together with a pharmaceutical carrier or excipient.

5. A DNA sequence coding for a protein as claimed in claim 1 or claim 2 and having the sequence:

```
(ATG) CAA ACC CAC AGC CTG GGT AGC AGG AGG ACC TTG ATG
CTC CTG GCA CAG ATG AGG AGA ATC TCT CTT TTC TCC TGC TTG
AAG GAC AGA CAT GAC TTT GGA TTT CCC CAG GAG GAG TTT GGC
AAC CAG TTC CAA AAG GCT GAA ACC ATC CCT GTC CTC CAT GAG
ATG ATC CAG CAG ATC TTC AAC CTC TTT ACC ACA AAA GAT TCA
TCT GCT GCT TGG GAT GAG GAC CTC CTA GAC AAA TTC TGC ACC
GAA CTC TAC CAG CAG CTG AAT GAC TTG GAA GCC TGT GTG ATG
CAG GAG GAG AGG GTG GGA GAA ACT CCC CTG ATG AAT GCG GAC
TCC ATC TTG GCT GTG AAG AAA TAC TTC CGA AGA ATC ACT CTC
TAT CTG ACA GAG AAG AAA TAC AGC CCT TGT GCC TGG GAG GTT
GTC AGA GCA GAA ATC ATG AGA TCC CTC TCT TTA TCA ACA AAC
TTG CAA GAA AGA TTA AGG AGG AAG GAA [TAA]
```

wherein the initiation codon ATG is enclosed in round brackets and a STOP-codon TAA is enclosed in square brackets;

and DNA sequences coding for a protein as claimed in claim 1 or claim 2 but differing from the above sequence according to the degeneracy of the genetic code.

6. A vector including and capable of expressing a DNA sequence as claimed in claim 5.

7. A host transformed with a vector as claimed in claim 6.

8. Process for the preparation of a vector as claimed in claim 6 or of a host as claimed in claim 7, which comprises:

(1) constructing a derivative of an alpha-1 interferon plasmid that has only one Eco RI site; (2) assembling a group of promoters ending in an ATG translation initiation codon and differing in the number of nucleotides between the "Shine-Dalgarno" sequence and this initiation codon; (3) joining this group of promoters to the amino-terminal coding sequence of the alpha-2 interferon gene; (4) isolating DNA fragments including the promoter regions ligated to the amino-terminal coding sequence of the alpha-2 interferon gene; (5) joining these promoter-containing fragments to the carboxy-terminal alpha-1 interferon coding sequence; and (6) transforming a host with the resulting plasmid and screening host clones for the production of active hybrid interferon.

9. Process for the preparation of a plasmid containing a gene coding for and capable of expressing the novel alpha-type interferon claimed in claim 1 or claim 2, and of a host transformed with such plasmid, which comprises:

a) Isolating from a plasmid containing the alpha-2 interferon gene a fragment extending from a restriction site preceding the gene to the Pvu II restriction site within that gene;

b) Annealing the coding strand of this fragment to a synthetic oligonucleotide, the sequence of which starts with the first nucleotide residue of the fifth codon of the mature alpha-2 interferon coding sequence;

c) Filling in the non-coding strand in the 5'→3'-direction and eating away the coding strand in the 3'→5'-direction and digesting the resulting double strand DNA fragment with Bgl II;

-25-

d) Digesting with Eco RI an alpha-1 interferon plasmid that has only one Eco RI site at a position upstream of the coding sequence for the alpha-1 interferon, filling it in and digesting it with Bgl II;

e) Ligating the larger fragment from this second plasmid to the amino terminal coding region of alpha-2 interferon DNA obtained in step c);

f) Transforming a suitable host; and

g) Modifying the plasmid to make the gene expressible.

10. Process for producing a novel interferon-type protein as claimed in any of claims 1 to 3, which comprises cultivating a host as claimed in claim 7.